(19) Europäisches Patentamt
European Patent Office
Office européen des brevets

(11) Veröffentlichungsnummer: **0 154 239**
**B1**

(12) # EUROPÄISCHE PATENTSCHRIFT

(45) Veröffentlichungstag der Patentschrift: **29.03.89**

(51) Int. Cl.⁴: **C 07 C 87/60**, C 07 C 85/00

(21) Anmeldenummer: **85101756.6**

(22) Anmeldetag: **18.02.85**

(54) **Verfahren zur Herstellung von 6-Chlor-2,4-dinitranilin.**

(30) Priorität: **01.03.84 DE 3407566**
**09.03.84 DE 3408631**

(43) Veröffentlichungstag der Anmeldung:
**11.09.85 Patentblatt 85/37**

(45) Bekanntmachung des Hinweises auf die Patenterteilung:
**29.03.89 Patentblatt 89/13**

(84) Benannte Vertragsstaaten:
**CH DE FR GB IT LI**

(56) Entgegenhaltungen:
**DD-A-206 150**
**DE-C-610 613**

**CHEMISCHES ZENTRALBLATT, Band 98, Nr. 15, 13. April 1927, Seiten 2063-2064, Berlin, DE; A. SEYEWETZ et al. "Über die Oxydation von Farbstoffen, zum Zweck ihrer Entfärbung, durch Natriumhypochlorit in der Kälte in saurem Medium"; & BULL. SOC. CHIM. DE FRANCE, Band 41, Nr. 4, Seiten 196-205 (Kat. A,D)**
**Seyewitz, Chaix; Bull. Sot. Chim. de France (4), 41, 197-204.**

(73) Patentinhaber: **HOECHST AKTIENGESELLSCHAFT, Postfach 80 03 20, D-6230 Frankfurt am Main 80 (DE)**

(72) Erfinder: **Arndt, Otto, Dr., Frankfurter Strasse 38, D-6238 Hofheim am Taunus (DE)**
Erfinder: **Papenfuhs, Theodor, Dr., Heinrich-Bleicher- Strasse 40, D-6000 Frankfurt am Main 50 (DE)**

**EP 0 154 239 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein vorteilhaftes Verfahren zur Herstellung von 6-Chlor-2,4-dinitranilin durch Chlorieren von 2,4-Dinitranilin mittels Natriumhypochlorit (NaOCl), bei welchen Mangel der bekannten Verfahren vermieden werden.

Die Herstellung von 6-Chlor-2,4-dinitranilin durch Chlorieren von 2,4-Dinitranilin ist an sich durch folgende in der Literatur beschriebene Verfahren bekannt:

1) Chlorierung von 2,4-Dinitranilin mit elementarem Chlor in Wasser bei 40 - 50° C in Gegenwart von Eisen (III)chlorid (IG-Farben DRP 610 613 (1933), Friedländer 21, 310);

2) Chlorierung von 2,4-Dinitranilin mit wäßriger Kaliumchloratlösung im Gemisch mit Salzsäure (Versl.Akad. Amsterdam 31, 294, Chemisches Zentralblatt 1923 III, 746, van de Vliet, Recueil des travaux chimiques des Pay-Bas, 43, 610);

3) Chlorierung von 2,4-Dinitranilin mit elementarem Chlor in Eisessig und konzentrierter Salzsäure (Chattaway, Dowden, J. Chem. Soc. 125, 1196);

4) Chlorierung von 2,4-Dinitranilin mit Natriumhypochlorit (NaOCl) unter Verwendung von 1 Äquivalent Salzsäure bei 15 - 20° C (Seyewitz, Chaix, Bulletin de la Société Chimique de France (4), 41, 197, 204).

Nach dem letztgenannten bekannten Verfahren erhält man ein 6-Chlor-2,4-dinitranilin, welches stark verunreinigt ist, was aus seinem viel zu niedrigen Schmelzpunkt von 145-146° C hervorgeht (Fp des reinen Produkts 158° C). Bei diesem bekannten Verfahren wird das während der Chlorierung successive freiwerdende Natriumhydroxid durch Zugabe einer entsprechenden Menge Salzsäure neutralisiert.

Das im 1. Literaturzitat beschriebene Verfahren hat gemäß Beispiel 2 der Deutschen Patentschrift 610 613 ein fein gemahlenes 2,4-Dinitranilin zur Voraussetzung und ist durch eine schlechte Raumzeitausbeute gekennzeichnet (Chlorierungsdauer 15 - 20 Stunden).

Das im vorstehend genannten 2. Literaturzitat beschriebene Verfahren führt zu einer hohen Abwasserbelastung mit Salzsäure.

Bei dem im genannten 3. Literaturzitat beschriebenen Verfahren erfordert die Regeneration der Essigsäure einen zusätzlichen größeren technischen Aufwand.

Es wurde gefunden, daß die den bekannten Verfahren anhaftenden Mängel (schlechte Produktqualität, zu leistender technischer Aufwand zur Erzielung einer Feinverteilung des Ausgangsprodukts, schlechte Raumzeitausbeute, hohe Abwasserbelastung durch anorganische Salze, zu leistender technischer Aufwand zur Regenerierung von angewandtem organischen Lösemittel) bei der Herstellung von 6-Chlor-2,4-dinitranilin durch Chlorieren von 2,4-Dinitranilin mit Natriumhypochlorit in Wasser unter Verwendung von Säure vermieden werden können, indem man die Chlorierung in Gegenwart eines Überschusses von Mineralsäure oder starker organischer Säuren, bezogen auf eingesetztes 2,4-Dinitranilin, und zwar bei einem pH-Wert < 0 und in Gegenwart eines Dispergiermittels bei Temperaturen von etwa 40 bis 50° C vornimmt und nach beendeter Chlorierung die angefallene saure wäßrige Suspension entweder (a) heiß filtriert und das abfiltrierte Reaktionsprodukt mit heißer wäßriger Alkalilösung, zweckmäßigerweise Sodalösung, wäscht oder (b) mit wäßriger Alkalilösung, zweckmäßigerweise Natronlauge, zunächst alkalisch stellt, heiß filtriert und das abfiltrierte Reaktionsprodukt anschließend mit heißer verdünnter Mineralsäure wäscht.

An Mineralsäuren kommen in erster Linie die technischen Mineralsäuren, wie Salzsäure (30 - 38 %-ig), Schwefelsäure, Phosphorsäure oder Salpetersäure, und als starke organische Säuren beispielsweise Ameisensäure oder Trichloressigsäure in Betracht. Vorzugsweise wird Salzsäure (30-31 %-ig) eingesetzt und zwar in einer Menge von 1,5 - 4 Äquivalenten HCl, bezogen auf eingesetztes 2,4-Dinitranilin. Es ist aber auch möglich und besonders wirtschaftlich, die beim vorliegenden Verfahren gegebenenfalls anfallende Waschsäure (im Sinne eines recycling) zur Einstellung des pH-Wertes < 0 einzusetzen.

Die Chlorierung des aromatischen Kohlenwasserstoffs (ArH) mittels Natriumhypochlorit erfolgt bekanntlich nach dem Reaktionsschema

$$ArH + NaOCl + ArCl + NaOH$$

Wird verfahrensgemäß beispielsweise nur 1 Äquivalent Salzsäure - bezogen auf die eingesetzte Menge an 2,4-Dinitranilin eingesetzt, welches gerade zur Neutralisation des 1 Äquivalents gebildeter NaOH ausreichen würde, so erhält man eine stark orangefarbene Suspension und das daraus isolierte Produkt füllt in schlechter Qualität und Ausbeute an. Gemäß J. Chem. Soc. 123, 2791 resultieren hierbei teilweise Azobenzole, gegebenenfalls über die N-Chlor-Verbindungen, wie beispielsweise 6,6'-Dichlor-2,4,2',4'-tetranitroazobenzol. Im Zuge eigener Untersuchungen wurden auch 2,4-Dinitrochlorbenzol und 1,2-Dichlor-3,5-dinitrobenzol als Nebenprodukte gefunden.

Durch den erfindungsgemäß zur Anwendung gelangenden Überschuß an Säure (pH-Wert des Reaktionsmediums < 0) wird auch ein Kontakt des zu chlorierenden 2,4-Dinitranilins mit unterchloriger Saure (HOCl) oberhalb pH 0 vermieden, welcher schädlich wäre.

Nachdem den eingangs genannten bekannten Verfahren der Chlorierung von 2,4-Dinitranilin zum 6-Chlor-2,4-dinitranilin die geschilderten schwerwiegenden Nachteile anhaften, und ferner entgegen der im 4. Literaturzitat (Seyewitz l.c.) aufgestellten Behauptung, daß außer der Kernchlorierung keine Nebenreaktionen ablaufen, sich nach unseren Beobachtungen sehr

wohl Nebenprodukte bilden, die schon in Spuren die Qualität des Endprodukts wegen ihrer starken Farbintensität negativ beeinflussen, muß es als überraschend erachtet werden, daß durch das erfindungsgemäße Verfahren das 6-Chlor-2,4-dinitranilin unter Vermeidung der Nachteile der bekannten Verfahren in hoher Ausbeute und hoher Reinheit hergestellt werden kann.

An das einzusetzende Dispergiermittel ist die Anforderung zu stellen, daß es gegenüber den Bedingungen der verfahrensgemäßen Chlorierung relativ stabil ist. Geeignete Dispergiermittel sind beispielsweise Verbindungen aus der Gruppe der Alkylsulfonate, Aralkylsulfonate (Alkylbenzolsulfonate und Alkylnaphthalinsulfonate), primären Alkylsulfate (Fettalkoholsulfate, Fettalkylsulfate, Fettalkoholsulfonate), sekundären Alkylsulfate, Fettsäurekondensationsprodukte (Kondensate mit aminogruppenhaltigen Körpern, Kondensate mit oxygruppenhaltigen Körpern, Kondensate mit aromatischen Kohlenwasserstoffen), Polyglykoläther oder Pyridinbasen. Hinsichtlich der einzusetzenden Menge ist es zweckmäßig, etwa 5 - 15 Gewichtsteile Dispergiermittel pro Mol 2,4-Dinitranilin anzuwenden.

Nachstehend seien noch Einzelheiten bzw. bevorzugte Ausführungsformen des erfindungsgemäßen Verfahrens beschrieben:

Zur Vorbereitung der Chlorierung wird das 2,4-Dinitranilin in der 5-10-fachen Wassermenge verrührt unter Zusatz eines Dispergiermittels, beispielsweise eines sekundären Alkansulfonats, wobei an das Dispergiermittel die Anforderung zu stellen ist, daß es relativ stabil gegenüber den Chlorierungsbedingungen ist. Zu der Suspension läßt man dann die Säure, vorzugsweise 31 %-ige Salzsäure, zulaufen. Eine Anwendung von mehr als 4 Äquivalenten Salzsäure wäre unzweckmäßig, weil dadurch eine erhöhte Belastung des Abwassers verursacht werden würde. Außerdem nehmen mit wachsender Säurekonzentration Nebenreaktionen zu, bei denen die Nitro- und Aminogruppen gegen Chlor ausgetauscht werden. Die Anwendung des Natriumhypochlorits (NaOCl) erfolgt in Form der Chlorbleichlauge. Unter Chlorbleichlauge versteht man wäßrige Lösungen von Natriumhypochlorit und Natriumchlorid, die durch Einleiten von Chlor in kalte wäßrige Natronlauge hergestellt werden können (Eine 13,5 gewichtsprozentige Chlorbleichlauge enthält 166 g wirksames Chlor auf 1229 g (= 1 Liter) entsprechend 2,34 Mol NaOCl auf 1 Liter) (Gmelins Handbuch der anorg.Chemie 6 (1927), Seite 293; Ullmanns Encyklopädie der techn. Chemie 9, 4. Aufl. (1975), Seite 544).

Es ist zweckmäßig, die Suspension des 2,4-Dinitranilins vor der Chlorierung auf Temperaturen von 40° - 50° C zu erwärmen und das Reaktionsgemisch während der Chlorierung auf diesem Temperaturniveau zu halten. Bei tieferen Temperaturen würde sich die unterchlorige Säure anreichern und unerwünschte Nebenreaktionen einleiten (Orangefärbung) bzw. schneller in Sauerstoff und Salzsäure zerfallen als nutzbringend abreagieren. Höhere Temperaturen als 50° C sind nicht zweckmäßig wegen der Förderung des Zerfalls der unterchlorigen Säure und wegen der Zersetzungsgefahr des- 6-Chlor-2,4-dinitranilins (sicherheitstechnische Gründe).

Das in Form der Chlorbleichlauge eindosierte Natriumhypochlorit soll möglichst sofort mit dem 2,4-Dinitranilin reagieren. Deshalb ist eine Mindesttemperatur von etwa 40° C erforderlich. Ferner ist es zu diesen Zweck vorteilhaft, die Chlorbleichlauge nicht zu rasch zulaufen zu lassen. Eine Akkumulierung der Chlorbleichlauge bei der Zudosierung zum Reaktionsgemisch ist zu vermeiden, was erreicht werden kann durch Beschränkung der Zutropfgeschwindigkeit der Chlorbleichlauge und durch ausreichendes Rühren des Reaktionsgemisches beim Zutropfen der Chlorbleichlauge.

Die optimale Dauer der Chlorierung beträgt ca. 8 Stunden. Längere Zeiten sind wegen der dadurch bedingten Herabsetzung der Raumzeitausbeute unwirtschaftlich. Bei kürzeren Zeiten als 4 Stunden treten Nebenreaktionen auf, die an einer Verfärbung der Suspension nach Orange und Dunkelfärbung der vereinigten Mutterlaugen und Waschfiltrate leicht zu erkennen sind. Bei optimaler Einhaltung von Temperatur, Säuremenge und Reaktionszeit erreicht man schon mit Natriumhypochlorit-Mengen von nur wenig mehr als 1 Äquivalent (beispielsweise 120 Mol %, bezogen auf das 2,4-Dinitranilin) einen vollständigen Umsatz des 2,4-Dinitranilins zum 6-Chlor-2,4-dinitranilin. Bei ungünstigen Bedingungen können selbst wesentlich größere Überschüsse von Natriumhypochlorit nutzlos sein. Es ist zweckmäßig, das Natriumhypochlorit in einer Menge von 1 bis 1,5 Äquivalenten, vorzugsweise von 1,2 Äquivalenten, bezogen auf das zu chlorierende 2,4-Dinitranilin, anzuwenden.

Weiterhin ist es vorteilhaft, nach Zugabe einer einem Äquivalent Natriumhypochlorit entsprechenden Menge Chlorbleichlauge den weiteren Zulauf der Chlorbleichlauge zunächst zu unterbrechen und ca. 1 - 2 Stunden unter langsamem Anheben der Temperatur auf 50° C nachzurühren. Dann läßt man die restliche Menge Chlorbleichlauge (ca. 0, 1 - 0,2 Mol) zulaufen, gegebenenfalls nach vorangegangener erneuter Zugabe von 1 Äquivalent Säure, beispielsweise 31 %-iger Salzsäure, und von Dispergiermittel. Nach dünnschichtchromatographischer Überprüfung des Endes des Umsatzes wird gegebenenfalls noch vorhandenes restliches aktives Chlor mit einer äquivalenten Menge 40 %-iger Natriumhydrogensulfitlauge zerstört. Die hellgelbe Suspension wird auf 60 - 70° C erwärmt. Die Erwärmung dient nur zur Vorbereitung der Heißfiltration.

Die Heißfiltration wird bei 70° C ausgeführt. Daran schließt sich eine Heißwäsche mit Wasser, 5 %-iger Sodalösung und erneut Wasser an. Der Sodaablauf ist zunächst schwarzbraun und wird

dann hellgelb, ein Zeichen, daß durch die Wäsche in wirksamer Weise stark färbende Nebenkomponenten entfernt werden. Bei diesem Verfahren zur Isolierung des 6-Chlor-2,4-dinitroanilins aus seinem Reaktionsgemisch werden zwar sehr wirkungsvoll die stark färbenden Nebenkomponenten entfernt, wodurch aber nicht ausgeschlossen wird, daß sich im Nutschgut lokale "alkalische Nester" bilden, die beim eventuellen Trocknen des Produkts zu lokalen Zersetzungsstellen führen, erkennbar an der unterschiedlichen Lösungsfarbe der an verschiedenen Stellen des Trockenguts entnommenen Proben (in z. B. Dimethylformamid: entweder hellorangegelb oder dunkelbraun).

Dieser Nachteil läßt sich wirkungsvoll ausschließen, wenn man die heiße Suspension vor der Heißfiltration mit konzentrierter Natronlauge auf pH 9 stellt. Man erhält jetzt bei der Filtration dunkelbraune Mutterlaugen, wobei das abfiltrierte Produkt jedoch hell bleibt. Die Heißwäsche wird dann mit Wasser (gegebenenfalls unter Zusatz von Dispergiermittel), dann mit verdünnter Mineralsäure, beispielsweise 2 %-iger Salzsäure, und schließlich wieder mit Wasser ausgeführt. Das Produkt zeigt nach dem Trocknen bei z. B. 60° C keine lokalen Zersetzungsstellen mehr. Die Filtrationen können auf offener Nutsche ausgeführt werden. Eine Belästigung durch Chlor ist nicht gegeben.

Man erhält das 6-Chlor-2,4-dinitranilin in hoher Ausbeute (95 % der Theorie) und hoher Reinheit (mindestens 98,5 %) vom Schmelzpunkt 156 - 158° C.

Als Verunreinigungen sind enthalten: 2,4-Dinitranilin < 1 %, 2,6-Dichlor-4-nitranilin ⩽ 0,5 %, 2,4-Dinitrochlor-benzol < 0,5 %.

6-Chlor-2,4-dinitranilin ist ein wertvolles Zwischenprodukt für Farbstoffe, beispielsweise als Diazokomponente für eine Reihe von Dispersionsfarbstoffen (DE-OS-2 155 866, DE-OS-2 256 314, EP-0 064 221, EP-0 066 235, EP-0 073 414, JA-AS 72/33 481 und JA-OS 72/26 417).

Die nachstehenden Beispiele dienen zur Erläuterung des erfindungsgemäßen Verfahrens, ohne es darauf zu beschränken. Teile bedeuten Gewichtsteile.

**Beispiel 1**

185 Teile 2,4-Dinitranilin (1 Mol) in handelsüblicher technisch feuchter oder technisch trockener Form werden in 1000 Teile Wasser, dem 5 Teile eines Dispergiermittels (sekundäres Alkansulfonat) zugesetzt sind, bei ca. 25° C unter üblichen Rührbedingungen eingetragen. Dazu läßt man 347 Teile 31 %-ige Salzsäure (3 Mol) laufen. Die Suspension (pH < 0) wird anschließend auf 40° C erwärmt. Dann wird in mindestens 4, höchstens 6, vorzugsweise 4 1/2 - 5 Stunden ein erster Anteil von 565 Teilen 13,5 %-iger Chlorbleichlauge (entsprechend 1,07 Mol NaOCl = 1,07 Mol aktives Chlor) bei 40° C zugetropft. Anschließend wird in ca. 1 Stunde auf 50° C erwärmt. Dann läßt man 116 Teile 31 %-ige Salzsäure (1 Mol) zulaufen(pH ~ 0,5). Gegebenenfalls werden nochmals 2,5 Teile Dispergiermittel zugegeben. Darauf wird in mindestens 1, höchstens 3, vorzugsweise 2 Stunden der zweite Anteil von 70 Teilen 13,5 %-iger Chlorbleichlauge bei 50° C zugetropft. Anschließend wird in ca. 1 Stunde auf 60 - 65° C erwärmt und gegebenenfalls nochmals 2,5 Teile Dispergiermittel hinzugegeben. Die dünne, hellgelbe, feinkörnige Suspension wird mit 313 Teilen 33 %-iger Natronlauge von pH 0,1 auf pH 9,0 gestellt.

Dann wird bei 70° C auf einer Nutsche abgesaugt, wobei die Mutterlauge dunkelbraun ist. Anschließend wird nacheinander gewaschen mit

1000 Teilen Wasser von 70 - 75° C, dem 2,5 Teile Dispergiermittel zugesetzt sind,

2000 Teilen einer 0,2 %-igen Salzsäure bei 70 - 75° C und 1000 Teilen Wasser von 70 - 75° C.

Man erhält nach dem Trocknen bei 60° C 209 Teile 6-Chlor-2,4-dinitranilin mit einem Reingehalt von mindestens 98,5 % und einem Schmelzpunkt von 156 - 158° C, entsprechend einer Ausbeute von 95 % der Theorie, als rein gelbes Pulver, dessen Lösungsfarbe in Dimethylformamid (100 mg/10 ml DMF) hellorangegelb ist.

Das Abwasser (ca. 6200 Teile, pH 1,1) enthält nur ca. 9 Teile Nitroaromaten und kann nach entsprechender Vorbehandlung der biologischen Reinigung zugeführt werden.

Verwendet man anstelle der Salzsäure eine äquivalente Menge an konzentrierter Schwefelsäure oder Phosphorsäure und arbeitet im übrigen wie beschrieben, so gelangt man zum praktisch gleichen Ergebnis.

**Beispiel 2**

Man verfährt, wie in Beispiel 1 beschrieben, jedoch ohne Anwendung von verdünnter Salzsäure bei der Heißwäsche, also unter Bedingungen eines unvollständigen Auswaschens von Alkali. Man erhält ein Produkt, das nach dem Trocknen bei 60° C stellenweise bräunliche Zersetzung aufweist. Fp. 150 - 153° C.

**Beispiel 3**

Man verfährt, wie in Beispiel 1 beschrieben, jedoch mit dem Unterschied, daß die Suspension bei pH 0,1 filtriert wird. Die Mutterlauge ist hellgelbbraun. Das Nutschgut wird anschließend nacheinander mit

1000 Teilen Wasser bei 70 - 75° C (gegebenenfalls unter Zusatz von 2,5 Teilen Dispergiermittel),

2000 Teilen 5 %-iger Sodalösung bei 70 - 75° C und
1000 Teilen Wasser bei 70 - 75° C gewaschen.
Der Filtratablauf der Sodawäsche ist anfangs dunkelbraun, am Ende hellgelb. Man erhält das Produkt in gleicher Ausbeute wie gemäß Beispiel 1, wobei die Lösungsfarbe des Feuchtprodukts in Dimethylformamid hellorangegelb ist. Nach dem Trocknen ist die Lösungsfarbe je nach dem Ort der Probenahme hellorangegelb oder dunkelbraun. Der Schmelzpunkt beträgt 150 - 155° C.

**Beispiel 4**

Man verfährt, wie in Beispiel 1 bechrieben, jedoch unter Anwendung der doppelten Menge Wasser bei der Chlorierung. Außerdem wird der erste Anteil von 565 Teilen Chlorbleichlauge innerhalb von 8 Stunden zugetropft (statt innerhalb von ca. 5 Stunden). Der zweite Anteil von 70 Teilen Chlorbleichlauge wird innerhalb von 2 Stunden zugetropft.

Die Aufarbeitung erfolgt, wie in Beispiel 3 beschrieben. Man erhält nach dem Trocknen bei 60° C 207 Teile 6-Chlor-2,4-dinitranilin mit einem Reingehalt von 98,5 % und einem Schmelzpunkt von 154 - 157° C. Die Ausbeute beträgt 94 % der Theorie. Beide Maßnahmen (Verdünnen und Verlängern der Chlorierungsdauer) führen zu keiner Verbesserung von Ausbeute und Qualität im Vergleich zur Arbeitsweise von Beispiel 1, sondern nur zu einer Verringerung der Raumzeitausbeute (= Raum- und Zeitaufwand pro Mengeneinheit Endprodukt).

**Beispiel 5**

Man verfährt, wie in Beispiel 4 beschrieben, jedoch mit dem Unterschied, daß die Suspension des 2,4-Dinitranilins vor der Chlorierung einer Naßvermahlung mit einem Hochleistungs-Dispergiergerät ausgesetzt wird. Man erhält dieselbe Ausbeute und Qualität wie bei der Arbeitsweise von Beispiel 4.

**Beispiel 6**

Man verfärt, wie in Beispiel 5 beschrieben, jedoch mit dem Unterschied, daß die Suspension des 2,4-Dinitranilins mit nur 173 Teilen 31 %-iger Salzsäure (1,5 Mol) angesetzt und dann auch später keine weitere Salzsäure mehr zugesetzt wird. Außerdem wird mit etwas mehr Chlorbleichlauge (= 850 Teile, entsprechend 1,6 Mol aktivem Chlor) chloriert.

Man erhält nur 190 Teile 6-Chlor-2,4-dinitranilin mit Schmelzpunkt 138 - 140° C. Aus dem Dünnschichtchromatogramm geht hervor, daß das Produkt noch erheblich mit 2,4-Dinitranilin verunreinigt ist. Daraus folgt, daß der Umsatz trotz erhöhten Einsatzes von Chlorbleichlauge unter diesen Bedingungen unvollständig ist. Das Produkt ist nicht hellgelb, sondern hellgelborange.

**Beispiel 7**

Man verfährt, wie in Beispiel 4 beschrieben, jedoch mit dem Unterschied, daß der erste Anteil der Chlorbleichlauge (= 565 Teile) in nur 4 Stunden und der 2. Anteil (= 70 Teile) in nur 1 Stunde zugetropft wird. Man erhält nach dem Trocknen bei 60° C nur 196 Teile 6-Chlor-2,4-dinitranilin vom Schmelzpunkt 145 - 155° C. Die Mutterlauge ist deutlich dunkler gefärbt als bei Beispiel 4.

**Beispiel 8** (Vergleichsbeispiel ohne Zusatz eines Dispergiermittels)

Man verfährt, wie in Beispiel 1 beschrieben, jedoch mit dem Unterschied, daß während des ganzen Verfahrenverlaufs kein Dispergiermittel zugesetzt wird. Nach beendeter Chlorierung ist die Suspension deutlich grobkörniger als die bei der Verfahrensweise des Beispiels 1 anfallende.

Nach der Heißfiltration bei pH 9, der üblichen Wäsche (jedoch ohne Anwendung eines Dispergiermittels) und nach dem Trocknen bei 60° C erhält man 197 Teile 6-Chlor-2,4-dinitranilin vom Schmelzpunkt 141 - 143° C (mit einem Gehalt von 12,6 % 2,4-Dinitranilin. Das erhaltene Produkt ist körnig und uneinheitlich gefärbt (gelb mit teilweise bräunlicher Verfärbung).

**Patentansprüche**

1. Verfahren zur Herstellung von 6-Chlor-2,4-dinitranilin durch Chlorieren von 2,4-Dinitranilin mit Natriumhypochlorit in Wasser unter Verwendung von Säure, dadurch gekennzeichnet, daß man die Chlorierung in Gegenwart einer Mineralsäure oder starken organischen Säure bei einem pH-Wert $< 0$ und in Gegenwart eines Dispergiermittels bei Temperaturen von etwa 40 bis 50° C durchführt und nach beendeter Chlorierung die angefallene saure wäßrige Dispersion entweder (a) heiß filtriert und das abfiltrierte Reaktionsprodukt mit heißer wäßriger Alkalilösung wäscht oder (b) mit wäßriger Alkalilösung zunächst alkalisch stellt, dann heiß filtriert und das abfiltrierte Reaktionsprodukt anschließend mit heißer verdünnter Mineralsäure wäscht.

2. Verfahren nach Anspruch 1, dadurch gekennzeichnet, daß man die Chlorierung in Gegenwart von 1,5 bis 4 Äquivalenten Salzsäure (HCl), bezogen auf eingesetztes 2,4-Dinitranilin durchführt.

## Claims

1. A process for the preparation of 6-chloro-2,4-dinitroaniline by chlorination of 2,4-dinitroaniline with sodium hypochlorite in water using acid, which comprises carrying out the chlorination in the presence of a mineral acid or strong organic acid at a pH value ⩽ 0 and in the presence of a dispersing agent at temperatures of about 40 to 50° and, when the chlorination has ended, either (a) filtering the resulting acid aqueous dispersion hot and washing the filtered reaction product with hot aqueous alkali solution, or (b) initially rendering the resulting acid aqueous dispersion alkaline with aqueous alkali solution and then filtering the mixture hot and subsequently washing the filtered reaction product with hot dilute mineral acid.

2. The process as claimed in claim 1, wherein the chlorination is carried out in the presence of 1.5 to 4 equivalents of hydrochloric acid (HCl), based on the 2,4-dinitroaniline employed.

## Revendications

1. Procédé pour préparer la chloro-6 dinitro-2,4 aniline par chloration de la dinitro-2,4 aniline au moyen de l'hypochlorite de sodium dans de l'eau en présence d'un acide, procédé caractérisé en ce qu'on effectue la chloration à des températures d'environ 40 à 50° C, en présence d'un acide minéral ou d'un acide organique fort, à un pH au plus égal à 0 et en présence d'un dispersant, et, après la chloration, ou bien (a) on filtre à chaud la dispersion aqueuse obtenue et on lave avec une solution aqueuse chaude d'un composé basique de métal alcalin le produit réactionnel séparé par filtration, ou bien (b) on alcalinise d'abord avec une solution aqueuse d'un composé basique de métal alcalin la dispersion aqueuse obtenue, puis on filtre à chaud et on lave ensuite avec un acide minéral dilué chaud le produit réactionnel séparé par filtration.

2. Procédé selon la revendication 1 caractérisé en ce qu'on effectue la chloration en présence de 1,5 à 4 équivalents d'acide chlorhydrique (HCl) par rapport à la dinitro-2,4 aniline mise en jeu.